# EUROPEAN PATENT SPECIFICATION

(11) **EP 1 679 090 B1**
(45) Date of publication and mention of the grant of the patent: **22.09.2010**
(21) Application number: 04762297.2
(22) Date of filing: 30.09.2004
(51) Int. Cl.: A61L 27/44, A61F 2/28

(54) **COMPOSITE BIOMATERIALS FOR BONE IMPLANTS**
ZUSAMMENGESETZTE BIOMATERIALIEN FÜR KNOCHENIMPLANTATE
BIOMATERIAUX COMPOSITES POUR IMPLANTS OSSEUX

(30) Priority: 16.10.2003 CU 2372003
(43) Date of publication of application: 12.07.2006
(73) Proprietor: Centro Nacional De Investigaciones Cientificas (CNIC), Ciudad de la Habana 12100 (CU)
(72) Inventor: GONZALEZ SANTOS, Ramon, La Lisa, Ciudad Habana 13 500 (CU); SUZARTE PAZ, Alberto Guillermo, Calle. Duany No. 3, Arroyo Naranjo. Ciudad Habana 13 800 (CU)
(74) Representative: Carpintero Lopez, Francisco
(86) International application number: PCT/CU2004/000009
(87) International publication number: WO 2005/035016

(56) References cited:
- WO-A1-00/71083
- US-A- 5 676 745
- US-B1- 6 414 050

## Description

This invention is applied in the field of synthesis and application of biomaterials, in particular those well-known as composed or mixed biomaterials ("composites") in this case formed by ceramic of calcium carbonate-phosphates and derivative polymers of the vinyl acetate and crotonic acid able to act as substitutes of the hard tissue when it has been damaged or lost or other biomedical applications as drug supports, controlled drug delivery systems, etc.

The need to substitute, reconstruct and/or regenerate the damaged or lost bone tissue in different places of the human body has been a challenge faced with more or less success along the centuries since the very beginning of humanity. Although one of the first solutions found was the use of different types of bone graft, nowadays they are being substituted by other biomaterials due to their diverse limitations, related mainly to the difficulty in their obtainment, trouble and surgical risks for the patients and variable effectiveness among others. The materials employed for these purposes make a long list that includes different types of metals, polymers, ceramic and glasses that are known generically as bone graft substitutes (Senn N. On the healing of aseptic cavities by implantation of antiseptic decalcified bone. Amer. J. Med. Sc., 98: 219-243; 1889*.,* Weber J.M., White E.W. it Carbonates minerals ace precursors of new ceramic, metal and polymer materials for biomedical applications. Min. Sci. Eng., 5: 151; 1973*,* Williams D.F. Challenges in materials for health care applications. Argew. Chem. Adv. Mater., 101 (5): 678; 1989*,* Hench L.L., Wilson T. Surface-activate biomaterials. Science, 226: 630; 1984*).* It can be said that until now the ideal biomaterial that suits all the exigency of the bone reconstructive surgery for different medical specialties has not been found, however, there is a widespread consent among the specialists, that from the point of view of their biocompatibility, tolerance by the human body and effectiveness in the cure, the calcium phosphates and in particular the hydroxyapatites are the most promissing biomaterials in this field (Klein C.P.A.T., Dreissen A.A., of Groot K., Van den Hooff A., Biodegradation behavior of various calcium phosphate materials in bone tissue. J. Biomed. Mater. Head., 17: 769-784; 1983*,* Shinazaki K., Mooney V., Comparative study of porous hydroxyapatite and calcium material phosphate in bone substitute. J. Orthop. Head., 3: 301; 1985*,* Damien C.J., Parsons J.R. Bone graft and bone graft substitutes: TO review of current technology and applications. J. Appl. Biomat., 2:187-208; 1991*,* Jallot E. Correlation between hydroxyapatite osseointegration and Young's Modulus. Med. Eng. Phys., 20: 697 ; 1998*).* It is it because these substances present a great chemical and structural identity with the mineral support of the bone, well-known as the "biological apatite".That is the reason why these compounds have become successful in the clinical use, mainly in the last 30 years *(*Cottrell D.A., Wolford L.M. Long-term evaluation of the uses of coralline hydroxyapatite in orthognatic surgery. J. Oral Maxillofac. Surg., 56: 935; 199*,.* Ayers R.A., Simske S.J., Nunes C.R., Wolford L.M. Long-term bone ingrowth and residual microhardeness of porous block hydroxyapatite implants in human. J. Oral Maxillofac. Surg., 56: 1297; 1998*,* González R., Blardoni F., Master H., Pereda O., Pancorbo E., Cienega M.A. Long-term results of the coralline porous hydroxyapatite HAP-200 ace bone implant's biomaterial in orthopedics and traumatology. CENIC Biological Sciences have, 32(2): 97-101; 2001*).*

But the research carried out by different specialists has allowed to check that the bone is formed by an inorganic support (approximately 65%) constituted mainly by these calcium phosphates above mentioned and the rest (35%) is organic matter and water. The organic phase is for the most part made up of collagen found in a close interrelation with the biological apatite (Weiner S., Traub W. Organization of hydroxyapatite crystals within collagen fibrils. FEBS, 206(2): 262; 1986).

Knowledge of the composition and structure of the bone tissue has stimulated the research and development of biomaterials of calcium phosphates with different composition, structure, porosities and with different biodegradation behaviours "in vivo" Nowadays there is a great variety of this type of implants among them those described in the patents US 4976736 (Dec., 1990), US 5900254 (May, 1999), FR 2776282 (Sept. 1999), US 6001394 (Dec. 1999), US 6331312 (Dec. 2001). Natural polymeric biomaterials (collagen, quitosane, cellulose, etc) as well as synthetic from different nature and origin, simulating the organic part of the bone have also been developed and applied with these purposes [US 5837752 (Nov. 1998), US 5919234 (Jul. 1999), US Patent Application 2003114552 (Jun. 2003)]. More recently an intense work on the production of composed or mixed materials (composite), made up by calcium phosphates and hydroxyapatite with different types of natural and synthetic polymers is being done with the purpose of achieving products with chemical, physical and mechanical properties more similar to the bone and with which a better functional acting as substitutes of the bone graft can be achieved.

A great number of combinations of this type of composed materials (composite) has been developed in the last years, among them can be mentioned the following: hydroxyapatite, collagen and a glycosaminoglycane (US 5071436, 1991), mixture of calcium phosphates with cellulose (FR2715853, 1995), hydroxyapatite with polylactic acid (W09746178, 1997), hydroxyapatite with silicone (US5728157, 1998), calcium phosphate with cellulose and its derivates (US6558709, 2003), several salts of calcium with several formulations of polymers (US6579532, 2003), hydroxyapatite, bone and several inorganic salts with polyethyleneglycol, waxes, hydrogels and acrylic latex (US 6605293, 2003).

A method for preparing a material for bone implants consisting of a porous outer coating and a hard inner body made of a material dispersed in a matrix is reported (WO 071083, 2000). The inner body is infused with different organic materials and between those vinyl acetate and not polymers derivates from vinyl acetate or crotonic acid are mentioned. Furthermore, neither there are any example of use of such polymers and behavior of these materials like systems of controlled drug release are not mentioned.

A composite material for bone implants including a mixture of a bio-functional polymer and a bio- functional substrate was prepared using a supercritical fluid (US 6 414 050, 2002). In a list of possible bio- functional polymers includes polyvinyl acetate and copolymers of vinyl alcohol and vinyl acetate, although these polymers are not mentioned in a specific example. The preparation and properties of such materials for applications as biomaterials under supercritical fluid are complicated and very expensive in order to guarantee the biocompatibility and non toxic requirements.

No report on possible combinations of derivative polymers of vinyl acetate and crotonic acid with any of the inorganic salts possible to be used as implant biomaterial was found.

Nevertheless, although the above products have shown good results in some medical applications, they don't still satisfy the diverse and growing need of the reconstructive surgery of the bones of different regions of the human body.

For some applications the surgeons prefer that the biomaterial to be implanted reabsorbs quickly leaving new bone in its place, in other cases it is demanded that the implant remains unchangeable for longer period of time according to the place and magnitude of the lesion to treat. Additionally it is very favorable if the biomaterial is able to deliver drugs in a controlled way, because it allows to treat different pathologies of the bone like infections, inflammatory processes, neoplacy, etc. at the same time.

On the other hand, many of the existing biomaterials for bone implants are not economically viable to apply them in mass population.

Recently derivative polymers from vinyl acetate and crotonic acid able to act as appropriate supports for the production of medicament of sustained action have been developed (Cuban Patents No. 22199 of 1993, 22880 of 2003, Int. Appl. PCT/CU99/00002 1999).

The present invention also involves new applications of these polymers in the production of implantable biomaterials in humans to reconstruct the bone tissue and for other therapeutic applications or reconstructive surgery.

The main objective of this invention is to obtain composed or mixed biomaterials ("composite") made up of calcium phosphates, carbonates and hydroxide, hydroxyapatite, carbonate-apatite or their mixtures in different proportions joined to derivative polymers from vinyl acetate and crotonic acid with appropriate properties to work as substitutes of bone implant.

Another objective of this invention is that the developed biomaterials can be dense or porous and with different biodegradation grades according to surgical necessities for the place, type of bone and magnitude of the lesion to treat.

We also search for that these biomaterials be formed by successive layers ceramic-polymer-ceramic or polymer-ceramic-polymer in such a way that surfaces of contact of the biomaterial with the live tissue can be obtained only formed by the polymer, by the ceramic or by both.

As application examples, clarifying the carrying out of our invention we give the following:
The developed biomaterials are made up of two types of compounds, an inorganic phase (A) and an organic phase (B).

The inorganic phase (A) it is constituted by calcium phosphates, hydroxide, carbonates, hydroxyapatite and carbonate-apatite or a mixture of them in different proportions as it is shown in the following chart:

**CHART 1. Composition of some mixtures of inorganic compounds (A) employed in the preparation of biomaterials.**

| Mixtures | Caa(PO4)b(H)c(CO3)d(OH)e (approximated value of the subindexes) | | | | | Molar ratio Ca/P |
|---|---|---|---|---|---|---|
| No. | a | b | c | d | e | |
| 1 | 3 | 2 | 0 | 0 | 0 | 1.5 |
| 2 | 10 | 6 | 0 | 0 | 2 | 1.66 |
| 3 | 10 | 6 | 0 | 1 | 0 | 1.66 |
| 4 | 96 | 59 | 4 | 9 | 1 | 1.62 |
| 5 | 100 | 3 | 0 | 95 | 1 | 33 |
| 6 | 44 | 33 | 16 | 2 | 1 | 1.3 |
| 7 | 9 | 5 | 1. | 1 | 2 | 1.8 |
| 8 | 9 | 4 | 2 | 2 | 2 | 2.25 |
| 9 | 10 | 3 | 0 | 5 | 1 | 3.3 |
| 10 | 9 | 6 | 2 | 0 | 2 | 1.5 |

The organic phase (B) is constituted by solutions polyvinyl acetate-co-crotonic acid of composition between 1 and 40% in weight of monomeric units of crotonic acid, monomer content between 0 and 100 ppm, molecular mass between 10 000 and 25 000 D and free from peroxides (CROTAV) or mixtures of them.

### EXAMPLE 5.

A homogeneous mixture of calcium salts with an approximate composition to the represented in No. 10 (chart 1) with a mean size of the particles of 0.1 mm, was moistened gradually with a solution of CROTAV (26%) in ethanol until obtaining a paste. The mixture was sieved to obtain particles between 1 and 2 mm of diameters and left to dry at room temperature. The dried granule was milled in a commiseration mill and the obtained powder was moistened again with ethanol, sieved again and dried to obtain a compact granule with mean size of the particles from 1 to 2 mm.

The product thus obtained with a content of approximately 15% of the polymer is appropriate as material for bone implant for the filling of cavities in the bone such as the sequels of tumors and cysts.

### EXAMPLE 6

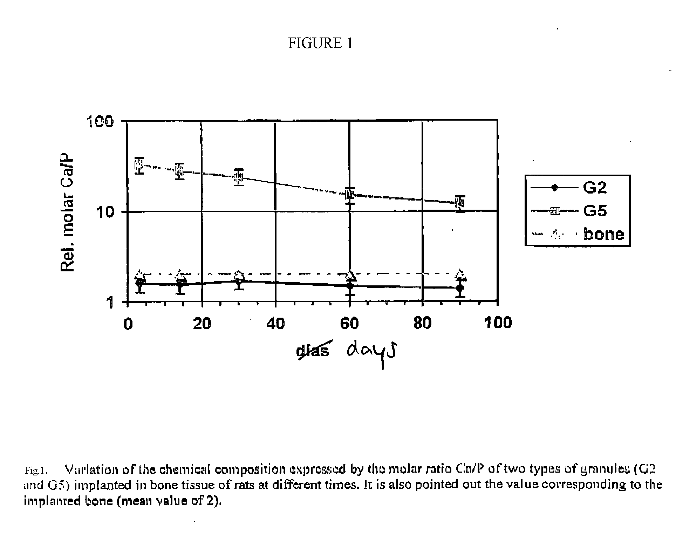

Two types of granule prepared according to the procedure described in example 5, one with a similar composition to that of the mixture 2 (G2) and another with that of the mixture 5 (G5) were implanted in bone tissue of rats (femur). The first on the left side and another on the right side after being sterilized with gamma rays at 25 Kgy. The implants were removed and analyzed at different times of postoperative evolution, being determined the variation in the composition of phases by means of FTIR spectroscopy and variation of molar ratio Ca/P by chemical analysis. It was found that while granule G2 did not change its composition considerably in 90 days, granule G5 degradated relatively quickly incorporating phosphorous to its structure to come closer to the half composition of the bone (Fig. 1).

The present invention presents the following advantages:
1- Physically and mechanically very stable biomaterials are obtained without the crumbling or loosening of isolated particles.
2- The obtained biomaterials can be dense or porous and they present appropriate biomechanical properties to work as bone graft substitutes.
3- Biomaterials with different degradation speeds in correspondence with the composition of the present phases are obtained.
4- The developed biomaterials also work as controlled drug delivery systems, with which is possible to restore the damaged or lost bone at the same time and to treat different bone pathologies with drugs like antibiotics, antiinflammatories, etc.
5- The obtained biomaterials have such porosity that they form a viable matrix for the growth of new tissue in their interior when they are implanted either in soft tissue or in the bone without forming "sack bottom"

## Claims

1. Composite biomaterials for bone implants having a multiphase chemical composition including an inorganic phase made of calcium salts and organic phase constituted by derivative polymers from vinyl acetate and crotonic acid.

2. Composite biomaterials for bone implants, according to claim 1 **characterized** for having an organic phase which can be homogeneous and evenly distributed in the whole volume of the solid and distributed all over the body of the inorganic support.

3. Composite biomaterials for bone implants, according to claim 1, **characterized in that** when the organic phase is covering the surface of the inorganic support, such surface is formed by successive layers ceramic-polymer-ceramic or polymer-ceramic-polymer in such a way that surfaces of contact of the biomaterial with the live tissue can be formed only by the polymer, by the ceramic or by both.

4. Composite biomaterials for bone implants, according to claim 1 **characterized** for having in their composition a proportion of the organic phase between 0.1 and 99%, which is constituted by poly-vinyl acetate-co-crotonic acid of composition between 1 and 40% in weight of monomeric units of crotonic acid, monomer content between 0 and 100 ppm, molecular mass between 10 000 and 25 000 Da (D) and free from peroxides or mixtures of them.

5. Composite biomaterials for bone implants, according to any of claims 1 to 4, suitable for their use as controlled drug delivery system when they are loaded with the corresponding drug in soft tissue as well as in a bone.

## Patentansprüche

1. Verbund-Biamaterialien für Knochenimplantate, die eine mehrphasige chemische Zusammensetzung aufweisen, einschließlich einer anorganischen Phase, die aus Calciumsalzen besteht, und einer organischen Phase, die gebildet ist aus Derivatpolymeren von Vinylacetat und Crotonsäure.

2. Verbund-Biomaterialien für Knochenimplantate nach Anspruch 1, **dadurch gekennzeichnet, dass** sie eine organische Phase aufweisen, die homogen und gleichförmig in dem gesamten Volumen des Feststoffs verteilt sein kann und die über den gesamten Körper des anorganischen Trägers verteilt ist.

3. Verbund-Biomaterialien für Knochenimplantate nach Anspruch 1, **dadurch gekennzeichnet, dass**, wenn die organische Phase die Oberfläche von dem anorganischen Träger bedeckt, die Oberfläche durch aufeinanderfolgende Schichten von Keramik-Polymer-Keramik oder Polymer-Keramik-Polymer derart gebildet wird, dass die Kontaktoberflächen von dem Biomaterial mit dem lebenden Gewebe nur durch das Polymer, nur durch die Keramik oder durch beide gebildet werden können.

4. Verbund-Biomaterialien für Knochenimplantate nach Anspruch 1, **dadurch gekennzeichnet, dass** sie in ihrer Zusammensetzung einen Anteil von der organischen Phase zwischen 0,1 und 99 % aufweisen, der durch Polyvinylacetat-co-Crotonsäure gebildet wird, mit einer Zusammensetzung zwischen 1 und 40 Gewichtsprozent Monomereinheiten von Crotonsäure, Monomergehalt zwischen 0 und 100 ppm, Molekülmasse zwischen 10.000 und 25.000 Da (D) und frei von Peroxiden oder Mischungen davon.

5. Verbund-Biomaterialien für Knochenimplantate nach einem der Ansprüche 1 bis 4, die für die Verwendung als kontrolliert freisetzendes Arzneimittelabgabesystem, wenn sie mit dem entsprechenden Arzneimittel beladen sind, sowohl in Weichgewebe als auch in einem Knochen geeignet sind.

## Revendications

1. Biomatériaux composites pour implants osseux ayant une composition chimique multiphase incluant une phase inorganique faite de sels de calcium et une phase organique constituée de polymères dérivés d'acétate de vinyle et d'acide crotonique.

2. Biomatériaux composites pour implants osseux selon la revendication 1, **caractérisés** comme ayant une phase organique qui peut être homogène et distribuée uniformément dans le volume total du solide et distribuée sur l'ensemble du support inorganique.

3. Biomatériaux composites pour implants osseux selon la revendication 1, **caractérisés en ce que** quand la phase organique recouvre la surface du support inorganique, une telle surface est formée de couches successives céramique-polymère-céramique ou polymère-céramique-polymère de sorte que les surfaces de contact du biomatériau avec le tissu vivant peuvent être formées seulement par le polymère, par la céramique ou par les deux.

4. Biomatériaux composites pour implants osseux selon la revendication 1, **caractérisés** comme ayant dans leur composition une proportion de phase organique entre 0,1 est 99%, qui est constituée par le poly(acétate de vinyle-co-acide crotonique) de composition entre 1 et 40 % en poids d'unités monomériques d'acide crotonique, de teneur en monomère entre 0 et 100 ppm, de masse moléculaire entre 10 000 et 25 000 Da (D) et exempt de peroxydes ou de mélanges de ceux-ci.

5. Biomatériaux composites pour implants osseux selon l'une quelconque des revendications 1 à 4, appropriés pour leur utilisation en tant que système de libération contrôlée de médicament quand ils sont chargés avec le médicament correspondant dans du tissu mou aussi bien que dans un os.
